# EUROPEAN PATENT SPECIFICATION

(11) **EP 3 740 205 B1**
(45) Date of publication and mention of the grant of the patent: **20.09.2023**
(21) Application number: 19751382.3
(22) Date of filing: 11.02.2019
(51) Int. Cl.: A61K 31/437, A61K 31/4162, A61P 35/00, A61P 31/04, A61P 31/00, A61P 37/00

(54) **METHODS OF SUPPRESSING MYELOID-DERIVED SUPPRESSOR CELLS IN PATIENTS**
VERFAHREN ZUR UNTERDRÜCKUNG VON MYELOIDABGELEITETEN SUPPRESSORZELLEN IN PATIENTEN
MÉTHODES DE SUPPRESSION DE CELLULES SUPPRESSIVES MYÉLOÏDES CHEZ DES PATIENTS

(30) Priority: 12.02.2018 US 201862629574 P
(43) Date of publication of application: 25.11.2020
(73) Proprietor: MediciNova, Inc., La Jolla, CA 92037 (US)
(72) Inventor: MATSUDA, Kazuko, La Jolla, California 92037 (US)
(74) Representative: Nederlandsch Octrooibureau
(86) International application number: PCT/US2019/017451
(87) International publication number: WO 2019/157428

(56) References cited:
- EP-B1- 1 106 178
- WO-A1-2007/142924
- WO-A1-2009/009529
- WO-A1-2013/188138
- WO-A1-2018/119262
- WO-A2-2010/095041
- KR-A- 20070 001 060
- US-A1- 2010 081 713
- US-A1- 2017 216 288
- KERRIE L. MCDONALD: "P01.20 Treatment of recurrent glioblastoma with the cytokine inhibitor, ibudilast in combination with temozolomide", NEURO-ONCOLOGY, 1 May 2017 (2017-05-01), page iii27, XP055631336, DOI: 10.1093/neuonc/nox036.096
- OTVOS BALINT ET AL: "Cancer Stem Cell-Secreted Macrophage Migration Inhibitory Factor Stimulates Myeloid Derived Suppressor Cell Function and Facilitates Glioblastoma Immune Evasion : CSC-Secreted MIF and MDSCs in GBM", STEM CELLS, vol. 34, no. 8, 27 May 2016 (2016-05-27), pages 2026-2039, XP055838385, ISSN: 1066-5099, DOI: 10.1002/stem.2393 Retrieved from the Internet: URL:https://api.wiley.com/onlinelibrary/td m/v1/articles/10.1002%2Fstem.2393>
- Juan Feng , Tatsuro Misu , Kazuo Fuijhara , Saburo Sakoda ,Yuji Nakatsuji , Hikoaki Fukaura , Seiji Kikuchi , Kunio Tashiro , Akio: "Ibudilast, a nonselective phosphodiesterase inhibitor, regulates Thl/Th2 balance and NKT cell subset in multiple sclerosis", Multiple Sclerosis Journal, vol. 10, no. 5, 1 October 2004 (2004-10-01), pages 494-498, XP009522786, ISSN: 1352-4585, DOI: 10.1191/1352458504ms1070oa
- Joanna Schwenkgrub; Malgorzata Zaremba; Ilona Joniec-Maciejak; Agnieszka Cudna; Dagmara Mirowska-Guzel; Iwona Kurkowska-Jastrz bsk: "The phosphodiesterase inhibitor, ibudilast, attenuates neuroinflammation in the MPTPmodel of Parkinson's disease", PLoS ONE, vol. 12, no. 7, 28 July 2017 (2017-07-28), pages 1-14, XP055630320, DOI: 10.1371/journal.pone.0182019

## Description

### CROSS-REFERENCE TO RELATED APPLICATIONS

This application claims the benefit of the priority date of U.S. Provisional Application No. 62/629,574, filed on February 12, 2018.

### BACKGROUND

The small molecule ibudilast (3-isobutyryl-2-isopropylpyrazolo[1,5-a]pyridine) is an inhibitor of macrophage inhibitory factor (MIF) (Cho et al., PNAS-USA, 2010 June 107: 11313-8), is a selective inhibitor of cyclic nucleotide phosphodiesterases (PDEs) 3A, 4, 10A1 and 11A1 (Gibson et al., Eur. J. Pharmacol., 538: 39-42, 2006), and has toll-like receptor-4 (TLR4) antagonistic activity (Yang et al., Cell Death and Disease (2016) 7, e2234; doi: 10.1038/cddis.2016.140). Ibudilast distributes well to the CNS (Sanftner et al., Xenobiotica, 2009 39: 964-977) and at clinically-relevant plasma or CNS concentrations, ibudilast selectively inhibits macrophage migration inhibitory factor (MIF) and, secondarily, PDEs 3, 4, 10 and 11. Ibudilast also acts as a leukotriene D4 antagonist, an anti-inflammatory, a PAF antagonist, and a vasodilatory agent (Thompson Current Drug Reports). Ibudilast is thought to exert a neuroprotective role in the central nervous system of mammals, presumably via suppression of the activation of glial cells (Mizuno et al., Neuropharmacology 46: 404-411, 2004).

Ibudilast has been widely used in Japan for relieving symptoms associated with ischemic stroke or bronchial asthma. In recent clinical trials, its use in the treatment of multiple sclerosis (MS), an inflammatory disease of the central nervous system, has been explored (News. Medical. Net; Pharmaceutical News, 2 Aug. 2005). As disclosed in this publication, this clinical trial was expected to treat "relapsing-remitting MS," however, no mention is made of progressive multiple sclerosis. In U.S. Patent No. 6,395,747, ibudilast is disclosed as a treatment for multiple sclerosis, which is generally understood to mean relapsing and remitting multiple sclerosis, not progressive multiple sclerosis. U.S. Patent Application Publication No. 20060160843 discloses ibudilast for the treatment of intermittent and short term pain, however, this is not pain related to a progressive neurodegenerative disease. However, U.S. Patent No. 9,314,452 discloses ibudilast as a treatment for amyotrophic lateral sclerosis, a progressive neurodegenerative disease. Similarly, U.S. Patent No. 8,138,201 discloses ibudilast as a treatment for primary progressive multiple sclerosis and/or secondary progressive multiple sclerosis. WO 2010/095041 discloses a combination of a neuraminidase inhibitor (oseltamivir) and ibudilast for use in the treatment of influenza infection.

While the use of ibudilast for a number of varying indications has been reported to date, to the best of the inventors' knowledge, its use in suppressing myeloid-derived suppressor cells (MDSCs) and reducing immune suppression has heretofore remained largely unexplored.

### SUMMARY

Note that the references to methods of treatment in this description are to be interpreted as references to the compounds, pharmaceutical compositions and medicaments of the present invention for use in a method for treatment of the human (or animal) body by therapy.

In one non-claimed aspect, provided herein is a method of suppressing myeloid-derived suppressor cells (MDSCs) in a patient diagnosed with cancer or suffering therefrom, the method comprising administering to the patient a therapeutically effective amount of ibudilast, or a pharmaceutical salt thereof.

In another non-claimed aspect, provided herein is a method of reducing immune suppression in a patient diagnosed with cancer or suffering therefrom, the method comprising administering to the patient a therapeutically effective amount of ibudilast, or a pharmaceutical salt thereof.

In another non-claimed aspect provided herein is a method of reducing regulatory T-cell count in a patient diagnosed with cancer or suffering therefrom, the method comprising administering to the patient a therapeutically effective amount of ibudilast, or a pharmaceutical salt thereof.

In another non-claimed aspect provided herein is a method of increasing CD4+ T-cell count in a patient diagnosed with cancer or suffering therefrom, the method comprising administering to the patient a therapeutically effective amount of ibudilast, or a pharmaceutical salt thereof.

In some embodiments, the cancer is a cancer of the circulatory system selected from angiosarcoma, fibrosarcoma, rhabdomyosarcoma, liposarcoma, myxoma, rhabdomyoma, fibroma, lipoma and teratoma, cancer of the mediastinum and pleura, or a vascular tumor; a cancer of the respiratory tract selected from cancer of the nasal cavity and middle ear, cancer of accessory sinuses, cancer of the larynx, cancer of the trachea, cancer of the bronchus and lung, small cell lung cancer (SCLC), non-small cell lung cancer (NSCLC), bronchogenic carcinoma, squamous cell carcinoma, undifferentiated small cell carcinoma, undifferentiated large cell carcinoma, adenocarcinoma, alveolar (bronchiolar) carcinoma, bronchial adenoma, sarcoma, lymphoma, chondromatous hamartoma or mesothelioma; a cancer of the gastrointestinal system selected from squamous cell carcinoma, adenocarcinoma, leiomyosarcoma, lymphoma, carcinoma, leiomyosarcoma, ductal adenocarcinoma, insulinoma, glucagonoma, gastrinoma, carcinoid tumors, vipoma, adenocarcinoma, carcinoid tumors, Karposi's sarcoma, leiomyoma, hemangioma, lipoma, neurofibroma, fibroma, adenocarcinoma, tubular adenoma, villous adenoma, hamartoma, or leiomyoma; a cancer of the genitourinary tract selected from adenocarcinoma, Wilm's tumor (nephroblastoma), lymphoma, leukemia, squamous cell carcinoma, transitional cell carcinoma, adenocarcinoma, adenocarcinoma, sarcoma of the prostate, seminoma, teratoma, embryonal carcinoma, teratocarcinoma, choriocarcinoma, interstitial cell carcinoma, fibroma, fibroadenoma, adenomatoid tumors, or lipoma; a cancer of the liver selected from hepatoma (hepatocellular carcinoma), cholangiocarcinoma, hepatoblastoma, angiosarcoma, hepatocellular adenoma, hemangioma, pheochromocytoma, insulinoma, vasoactive intestinal peptide tumor, islet cell tumor or glucagonoma; a cancer of the bone selected from osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma or giant cell tumors; a cancer of the nervous system selected from primary CNS lymphoma, osteoma, hemangioma, granuloma, xanthoma, osteitis deformans, meningioma, meningiosarcoma, gliomatosis, astrocytoma, medulloblastoma, glioma, ependymoma, germinoma (pinealoma), oligodendroglioma, schwannoma, retinoblastoma, congenital tumors, spinal cord neurofibroma, meningioma, glioma, or sarcoma; a cancer of the reproductive system selected from endometrial carcinoma, cervical carcinoma, pre-tumor cervical dysplasia, ovarian carcinoma , serous cystadenocarcinoma, mucinous cystadenocarcinoma, unclassified carcinoma, granulosa-thecal cell tumors, Sertoli-Leydig cell tumors, dysgerminoma, malignant teratoma, squamous cell carcinoma of the vulva, intraepithelial carcinoma of the vulva, adenocarcinoma of the vulva, fibrosarcoma of the vulva, melanoma of the vulva, vaginal clear cell carcinoma, vaginal squamous cell carcinoma, vaginal botryoid sarcoma (embryonal rhabdomyosarcoma), carcinoma of the fallopian tubes placental cancer, penile cancer, prostate cancer, or testicular cancer; a cancer of the hematologic system selected from myeloid, acute lymphoblastic leukemia, chronic lymphocytic leukemia, myeloproliferative diseases, multiple myeloma, myelodysplastic syndrome, Hodgkin's disease, or non-Hodgkin's lymphoma; a cancer of the oral cavity selected from lip cancer, tongue cancer, gum cancer, floor of mouth cancer, palate cancer, parotid gland cancer, salivary gland cancer, tonsil cancer, cancer of the oropharynx, cancer of the nasopharynx, pyriform sinus cancer, or cancer of the hypopharynx; a cancer of the skin selected from malignant melanoma, cutaneous melanoma, basal cell carcinoma, squamous cell carcinoma, Karposi's sarcoma, moles dysplastic nevi, lipoma, angioma, dermatofibroma or keloidal cancer; or a cancer selected from cancer of the adrenal glands, neuroblastoma, cancer of connective and soft tissue, cancer of the retroperitoneum and peritoneum, eye cancer, intraocular melanoma, cancer of adnexa, breast cancer, head or/and neck cancer, anal cancer, thyroid cancer, parathyroid cancer, cancer of the adrenal gland , cancer of the endocrine glands and related structures, secondary and unspecified malignant neoplasm of lymph nodes, secondary malignant neoplasm of respiratory and digestive systems or secondary malignant neoplasm of other sites. In some embodiments, the cancer is glioblastoma multiforme (GBM). In some embodiments, the cancer is not glioblastoma multiforme (GBM).

The present invention provides a method of suppressing myeloid-derived suppressor cells (MDSCs) in a patient diagnosed with microorganism infection or suffering therefrom, the method comprising administering to the patient a therapeutically effective amount of ibudilast, or a pharmaceutical salt thereof. In some embodiments, suppression of MDSCs reduces immune suppression in the patient. In some embodiments, suppression of MDSCs increases CD4 T-cell count in the patient.

In another aspect of the invention, provided herein is a method of reducing immune suppression in a patient diagnosed with microorganism infection or suffering therefrom, the method comprising: administering to the patient a therapeutically effective amount of ibudilast, or a pharmaceutical salt thereof.

In another aspect of the invention, provided herein is a method of reducing regulatory T-cell count in a patient diagnosed with microorganism infection or suffering therefrom, the method comprising administering to the patient a therapeutically effective amount of ibudilast, or a pharmaceutical salt thereof.

In another aspect of the invention, provided herein is a method of increasing CD4+ T-cell count in a patient diagnosed with microorganism infection or suffering therefrom, the method comprising administering to the patient a therapeutically effective amount of ibudilast, or a pharmaceutical salt thereof.

In some embodiments, the microorganism infection is caused by virus, bacteria, fungus, or any combination of two or more thereof.

In a non-claimed aspect, provided herein is a method of suppressing myeloid-derived suppressor cells (MDSCs) in a patient diagnosed with sepsis or suffering therefrom, the method comprising administering to the patient a therapeutically effective amount of ibudilast, or a pharmaceutical salt thereof. In some embodiments, suppression of MDSCs reduces immune suppression in the patient. In some embodiments, suppression of MDSCs increases CD4 T-cell count in the patient.

In another non-claimed aspect, provided herein is a method of reducing immune suppression in a patient diagnosed with sepsis or suffering therefrom, the method comprising administering to the patient a therapeutically effective amount of ibudilast, or a pharmaceutical salt thereof.

In another non-claimed aspect, provided herein is a method of reducing regulatory T-cell count in a patient diagnosed with sepsis or suffering therefrom, the method comprising administering to the patient a therapeutically effective amount of ibudilast, or a pharmaceutical salt thereof.

In another non-claimed aspect, provided herein is a method of increasing CD4+ T-cell count in a patient diagnosed with sepsis or suffering therefrom, the method comprising administering to the patient a therapeutically effective amount of ibudilast, or a pharmaceutical salt thereof.

In some embodiments, ibudilast, or the pharmaceutically acceptable salt thereof, is administered for at least 3 months. In some embodiments, ibudilast, or the pharmaceutically acceptable salt thereof, is administered for at least 6 months. In some embodiments, ibudilast, or the pharmaceutically acceptable salt thereof, is administered for at least one year. In some embodiments, ibudilast, or the pharmaceutically acceptable salt thereof, is administered for at least two years.

In some embodiments, ibudilast, or the pharmaceutically acceptable salt thereof, is administered at least once daily. In some embodiments, ibudilast, or the pharmaceutically acceptable salt thereof, is administered orally. In some embodiments, ibudilast, or the pharmaceutically acceptable salt thereof, is the only active agent administered to the patient.

In some embodiments, the therapeutically effective amount of ibudilast, or the pharmaceutically acceptable salt thereof, is from 0.1 mg to 720 mg per day. In some embodiments, the therapeutically effective amount of ibudilast, or the pharmaceutically acceptable salt thereof, is at least 30 mg/day. In some embodiments, the therapeutically effective amount of ibudilast, or the pharmaceutically acceptable salt thereof, is from 30 mg to 200 mg per day. In some embodiments, the therapeutically effective amount of ibudilast, or the pharmaceutically acceptable salt thereof, is 60 mg to 600 mg daily. In some embodiments, the therapeutically effective amount of ibudilast, or the pharmaceutically acceptable salt thereof, is 100 mg to 480 mg daily.

In some embodiments, the therapeutically effective amount of ibudilast, or the pharmaceutically acceptable salt thereof, is selected from the group consisting of 30 mg/day, 60 mg/day, 90 mg/day, 100 mg/day, 120 mg/day, 150 mg/day, 180 mg/day, 210 mg/day, 240 mg/day, 270 mg/day, 300 mg/day, 360 mg/day, 400 mg/day, 440 mg/day, 480 mg/day, 520 mg/day, 580 mg/day, 600 mg/day, 620 mg/day, 640 mg/day, 680 mg/day, and 720 mg/day.

In some embodiments, the therapeutically effective amount is administered as a single dose or is divided into two, three, or four doses.

### BRIEF DESCRIPTION OF THE FIGURES

FIG. 1 illustrates the GBM-derived co-culture used to induce MDSCs and conduct the test of FIG. 2.
FIG. 2 shows T-cell proliferation in the presence of MDSCs and MDSCs plus ibudilast.
FIG. 3 shows that ibudilast reduces M-MDSCs associated with tumor but not M-MDSCs associated with blood, spleen or bone marrow.

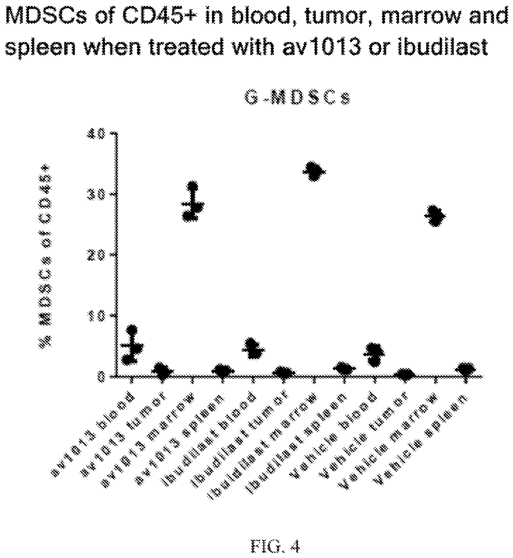

FIG. 4 compares MDSCs of CD45+ in blood, tumor, bone marrow and spleen when each of these matrices is exposed to ibudilast or AV1013.

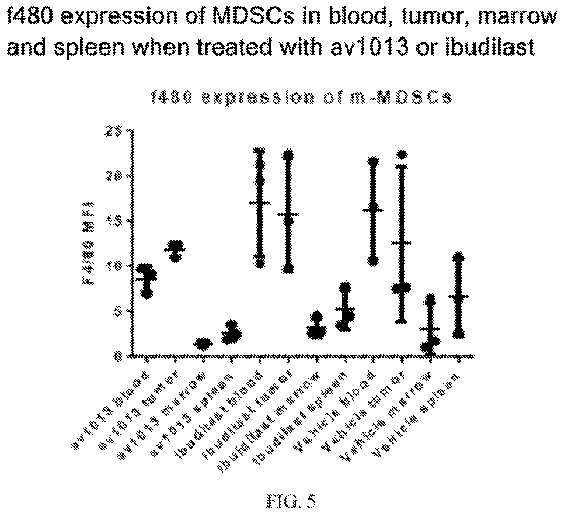

FIG. 5 shows f480 expression of MDSCs in blood, tumor, marrow and spleen when treated with AV 1013 or ibudilast.
FIG. 6 shows % T-regulatory cells of CD45+ in blood, tumor, marrow and spleen when treated with AV1013 or ibudilast.
FIG. 7 shows % CD8 of CD45+ in blood, tumor, marrow and spleen when treated with AV1013 or ibudilast.
FIG. 8 shows % CD4 of CD45+ in blood, tumor, marrow and spleen when treated with AV1013 or ibudilast.

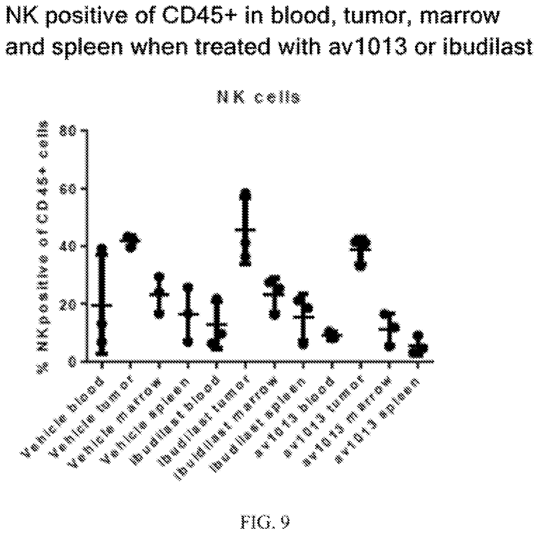

FIG. 9 shows % NK positive of CD45+ in blood, tumor, marrow and spleen when treated with AV 1013 or ibudilast.
FIG. 10 shows mRNA expression analysis of CD45+ cells isolated from n=3 vehicle-treated tumors and n=3 ibudilast-treated tumors.
FIG. 11 shows analysis of the MIF signaling axis in raw counts from CD45+ cells isolated from n=3 vehicle-treated tumors and n=3 ibudilast-treated tumors. (Unpaired T-test: p<0.01**).
FIG. 12 shows in vitro ibudilast treatment of co-culture for 24 hours led to reduced GL261 secretion of MCP-1(ccl2) in a dose dependent manner. (Unpaired T-test: p<0.01**, p<0.05*).
FIG. 13 shows reduction on MCP-1 according to analysis of serum from ibudilast-treated mice. (Unpaired T-test: p<0.01^{∗∗}).
FIG. 14 shows Kaplan Meier survival analysis of vehicle- versus ibudilast-treated mice (Vehicle (n=10, median=19 days); ibudilast (n=10, median=undetermined), log rank p=0. 0 16).
FIG. 15 shows diagram of co-culture set-up demonstrating the flow cytometry gating of Live/CD45+/CD11b+/GR-1 gating strategy post incubation.
FIG. 16 shows flow cytometry analysis of surface levels of CD74 and CXCR2 on FACs sorted M-MDSCs and G-MDSCs. (Unpaired T-test: p<0.001***).
FIG. 17 shows MDSC generation in co-cultures with MIF inhibitors quantified by flow cytometry. (Unpaired T-test: p<0.01**, p<0.05*).
FIG. 18 shows RNA sequencing of FACs sorted M-MDSCs, G-MDSCs, and CD11b+ cells from n=3 mice.
FIG. 19 shows ELISA assay of MIF levels in media 24 hours after seeding equal numbers of cells as utilized in the co-culture system. (Unpaired T-test: p<0.01**, p<0.05*).
FIG. 20 shows in vivo tumor bearing hemisphere tumor and non-tumor bearing hemisphere were analyzed by flow cytometry for M-MDSCs and G-MDSCs

### DETAILED DESCRIPTION

The practice of the present disclosure will employ, unless otherwise indicated, conventional methods of chemistry, biochemistry, and pharmacology, within the skill of the art. Such techniques are explained fully in the literature. See, e.g.; A. L. Lehninger, Biochemistry (Worth Publishers, Inc., current addition); Morrison and Boyd, Organic Chemistry (Allyn and Bacon, Inc., current addition); J. March, Advanced Organic Chemistry (McGraw Hill, current addition); Remington: The Science and Practice of Pharmacy, A. Gennaro, Ed., 20th Ed.; FDA's Orange Book, Goodman & Gilman The Pharmacological Basis of Therapeutics, J. Griffith Hardman, L. L. Limbird, A. Gilman, 11th Ed., 2005, The Merck Manual, 18th edition, 2007, and The Merck Manual of Medical Information 2003.

### Definitions

Before describing the present disclosure in detail, it is to be understood that this disclosure is not limited to particular administration modes, patient populations, and the like, as such may vary, as will be apparent from the accompanying description and figures.

It must be noted that, as used in this specification and the intended claims, the singular forms "a," "an," and "the" include plural referents unless the context clearly dictates otherwise. Thus, for example, reference to "a drug" includes a single drug as well as two or more of the same or different drugs, reference to "an optional excipient" refers to a single optional excipient as well as two or more of the same or different optional excipients, and the like.

In describing and claiming the present disclosure, the following terminology will be used in accordance with the definitions described below.

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of' shall mean excluding more than trace elements of other ingredients and substantial method steps. Embodiments defined by each of these transition terms are within the scope of this invention. When an embodiment is defined by one of these terms (e.g., "comprising") it should be understood that this disclosure also includes alternative embodiments, such as "consisting essentially of" and "consisting of" for said embodiment.

"Pharmaceutically acceptable excipient or carrier" refers to an excipient that may optionally be included in the compositions of the disclosure and that causes no significant adverse toxicological effects to the patient.

"Pharmaceutically acceptable salt" includes, but is not limited to, amino acid salts, salts prepared with inorganic acids, such as chloride, sulfate, phosphate, diphosphate, bromide, and nitrate salts, or salts prepared from the corresponding inorganic acid form of any of the preceding, e.g., hydrochloride, etc., or salts prepared with an organic acid, such as malate, maleate, fumarate, tartrate, succinate, ethylsuccinate, citrate, acetate, lactate, methanesulfonate, benzoate, ascorbate, para-toluenesulfonate, palmoate, salicylate and stearate, as well as estolate, gluceptate and lactobionate salts. Similarly salts containing pharmaceutically acceptable cations include, but are not limited to, sodium, potassium, calcium, aluminum, lithium, and ammonium (including substituted ammonium).

"Active molecule" or "active agent" as described herein includes any agent, drug, compound, composition of matter or mixture which provides some pharmacologic, often beneficial, effect that can be demonstrated in-vivo or in vitro. This includes foods, food supplements, nutrients, nutraceuticals, drugs, vaccines, antibodies, vitamins, and other beneficial agents. As used herein, the terms further include any physiologically or pharmacologically active substance that produces a localized or systemic effect in a patient. In specific embodiments, the active molecule or active agent may include ibudilast or a pharmaceutically acceptable salt thereof.

"Substantially" or "essentially" means nearly totally or completely, for instance, 95% or greater of some given quantity.

"Optional" or "optionally" means that the subsequently described circumstance may or may not occur, so that the description includes instances where the circumstance occurs and instances where it does not.

The terms "subject," "individual" or "patient" are used interchangeably herein and refer to a vertebrate, preferably a mammal. Mammals include, but are not limited to, mice, rodents, rats, simians, humans, farm animals, dogs, cats, sport animals and pets.

The terms "pharmacologically effective amount" or "therapeutically effective amount" of a composition or agent, as provided herein, refer to a nontoxic but sufficient amount of the composition or agent to provide the desired response, such as a reduction or reversal of progressive neurodegenerative diseases. The exact amount required will vary from subject to subject, depending on the species, age, and general condition of the subject, the severity of the condition being treated, the particular drug or drugs employed, mode of administration, and the like. An appropriate "effective" amount in any individual case may be determined by one of ordinary skill in the art using routine experimentation, based upon the information provided herein.

The term "about," will be understood by persons of ordinary skill in the art and will vary to some extent depending upon the context in which it is used. If there are uses of the term which are not clear to persons of ordinary skill in the art given the context in which it is used, "about" will mean up to plus or minus 10% of the particular term. For example, in some embodiments, it will mean plus or minus 5% of the particular term. Certain ranges are presented herein with numerical values being preceded by the term "about". The term "about" is used herein to provide literal support for the exact number that it precedes, as well as a number that is near to or approximately the number that the term precedes. In determining whether a number is near to or approximately a specifically recited number, the near or approximating unrecited number may be a number, which, in the context in which it is presented, provides the substantial equivalent of the specifically recited number.

As used herein, the terms "glioblastoma multiforme" or "glioblastoma" "or malignant glioma" are well-understood terms in the art. In some embodiments, "glioblastoma multiforme" or "glioblastoma" or "malignant glioma" are used interchangeably herein and refer to a brain tumor that arises from astrocytes. In some embodiments, glioblastoma is classical glioblastoma, proneural glioblastoma, mesenchymal glioblastoma or neural glioblastoma. In some embodiments, glioblastoma is classical glioblastoma.

As used herein, the term "treatment" or "treating" means any treatment of a disease or condition or associated disorder, in a patient, including inhibiting the disease or condition, that is, arresting or suppressing the development of clinical symptoms, such as cachexia in cancer; and/or relieving the disease or condition that is causing the regression of clinical symptoms, e.g., increasing overall survival or reducing tumor burden.

In some aspects, the term treating refers to an improvement in clinical outcomes. The term "clinical outcome" refers to any clinical observation or measurement relating to a patient's reaction to a therapy. Non-limiting examples of clinical outcomes include tumor response (TR), overall survival (OS), progression free survival (PFS), disease free survival, time to tumor recurrence (TTR), time to tumor progression (TTP), relative risk (RR), toxicity or side effect. "Overall Survival" (OS) intends a prolongation in life expectancy as compared to naive or untreated individuals or patients. "Progression free survival" (PFS) or "Time to Tumor Progression" (TTP) indicates the length of time during and after treatment that the cancer does not grow. Progression-free survival includes the amount of time patients have experienced a complete response or a partial response, as well as the amount of time patients have experienced stable disease. "Tumor Recurrence" as used herein and as defined by the National Cancer Institute is cancer that has recurred (come back), usually after a period of time during which the cancer could not be detected. The cancer may come back to the same place as the original (primary) tumor or to another place in the body. It is also called recurrent cancer. "Time to Tumor Recurrence" (TTR) is defined as the time from the date of diagnosis of the cancer to the date of first recurrence, death, or until last contact if the patient was free of any tumor recurrence at the time of last contact. If a patient had not recurred, then TTR was censored at the time of death or at the last follow-up. "Relative Risk" (RR), in statistics and mathematical epidemiology, refers to the risk of an event (or of developing a disease) relative to exposure. Relative risk is a ratio of the probability of the event occurring in the exposed group versus a non-exposed group.

"Treatment" or "treating" includes arresting the development of or reversing the symptom or symptoms of a disease. Non-limiting example of improvements in clinical outcome include longer survival time, reduction in tumor size, non-growth in tumor size, and/or lack of exacerbation in neurological symptoms. Non-limiting examples of neurological symptoms include double vision, vomiting, loss of appetite, changes in mood and personality, changes in ability to think and learn, seizures, speech difficulty, and cognitive impairment.

Other objects, features and advantages of the present disclosure will become apparent from the following detailed description. It should be understood, however, that the detailed description and the specific examples, while indicating specific embodiments of the disclosure, are given by way of illustration only, since various changes and modifications within the spirit and scope of the disclosure will become apparent to those skilled in the art from this detailed description.

The methods of the disclosure are based upon administration of the molecule, ibudilast. Ibudilast is a small molecule drug (molecular weight of 230.3) having the structure shown below.

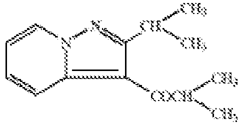

Ibudilast is also found under ChemBank ID 3227, CAS # 50847-11-5, and Beilstein Handbook Reference No. 5-24-03-00396. Its molecular formula corresponds to C14H18N2O. Ibudilast is also known by various chemical names including 2-methyl-1-(2-(1-methylethyl)pyrazolo(1,5-a)pyridin-3-yl)1-propanone; 3-isobutyryl-2-isopropylpyrazolo(1,5-a)pyridine; and 1-(2-isopropyl-pyrazolo[1,5-a]pyridin-3-yl)-2-methyl-propan-1-one. Other synonyms for ibudilast include Ibudilastum (Latin), BRN 0656579, KC-404, and MN-166. Its brand name is Ketas^{®}. Ibudilast, as referred to herein, is meant to include any and all pharmaceutically acceptable salt forms thereof, prodrug forms (e.g., the corresponding ketal), solvates, and the like, as appropriate for use in its intended formulation for administration.

Ibudilast is an inhibitor of the macrophage inhibitory factor (MIF). Ibudilast is also a selective inhibitor of cyclic nucleotide phosphodiesterases (PDEs) 3A, 4, 10A1 and 11A1 (Gibson et al., Eur J Pharmacol 538: 39-42, 2006)., and has also been reported to have leukotriene D4 and PAF antagonistic activities. Its profile appears effectively anti-inflammatory and unique in comparison to other PDE inhibitors and anti-inflammatory agents. PDEs catalyze the hydrolysis of the phosphoester bond on the 3'-carbon to yield the corresponding 5'-nucleotide monophosphate. Thus, they regulate the cellular concentrations of cyclic nucleotides. Since extracellular receptors for many hormones and neurotransmitters utilize cyclic nucleotides as second messengers, the PDEs also regulate cellular responses to these extracellular signals. There are at least eight classes of PDEs: Ca₂+/calmodulin-dependent PDEs (PDE1); cGMP-stimulated PDEs (PDE2); cGMP-inhibited PDEs (PDE3); cAMP-specific PDEs (PDE4); cGMP-binding PDEs (PDE5); photoreceptor PDEs (PDE6); high affinity, cAMP-specific PDEs (PDE7); and high affinity cGMP-specific PDEs (PDE9). Ibudilast acts to suppress inflammation via action on inflammatory cells (e.g., glial cells) resulting in the suppression of both pro-inflammatory mediator and neuroactive mediator release. Ibudilast may also suppress the production of pro-inflammatory cytokines (IL-1β, TNF-α) and may enhance the production of the anti-inflammatory cytokines (IL-4, IL-10). References related to the foregoing include the following: Obernolte, R., et al. (1993) "The cDNA of a human lymphocyte cyclic-AMP phosphodiesterase (PDE IV) reveals a multigene family" Gene 129: 239-247; Rile, G., et al. (2001) "Potentiation of ibudilast inhibition of platelet aggregation in the presence of endothelial cells" Thromb. Res. 102: 239-246; Souness, J. E., et al. (1994) "Possible role of cyclic AMP phosphodiesterases in the actions of ibudilast on eosinophil thromboxane generation and airways smooth muscle tone" Br. J. Pharmacol. 111: 1081-1088; Suzumura, A., et al. (1999) "Ibudilast suppresses TNF.alpha, production by glial cells functioning mainly as type III phosphodiesterase inhibitor in CNS" Brain Res. 837: 203-212; Takuma, K., et al. (2001) "Ibudilast attenuates astrocyte apoptosis via cyclic GMP signaling pathway in an in vitro reperfusion model" Br. J. Pharmacol. 133: 841-848. With regards to the treatment of cancers of the CNS, ibudilast exhibits good CNS penetration. (Sanftner et al Xenobiotica 2009 39: 964-977).

Without being bound to any one particular theory, the efficacy of ibudilast to suppress myeloid-derived suppressor cells (MDSCs); reduce immune suppression; reduce regulatory T-cell count; or increase CD4+ T-cell count; or any combination of two or more thereof, in a patient diagnosed with cancer or suffering therefrom may not be due to its MIF inhibitory activity, but rather due to ibudilast's interaction with other known or unknown targets (such as, but not limited to, one or more PDEs and/or TLR4) along with or regardless of ibudilast's MIF inhibitory activity.

As stated previously, a reference to any one or more of the herein-described drugs, in particular ibudilast, is meant to encompass, where applicable, any and all enantiomers, mixtures of enantiomers including racemic mixtures, pharmaceutically acceptable salt forms, hydrates (e.g., monohydrates, dihydrates, etc.), solvates, different physical forms (e.g., crystalline solids, amorphous solids).

### METHODS OF ADIMINSTRATION

As set forth above, in one aspect, the present invention is directed to a methods of suppressing myeloid-derived suppressor cells (MDSCs); reducing immune suppression; reducing regulatory T-cell count; and increasing CD4+ T-cell count, in a patient diagnosed with or suffering from microorganism infection, the method comprising administering to the patient a therapeutically effective amount of ibudilast, or a pharmaceutical salt thereof. Such administration is effective to promote immune response to the microorganism infection, and result in attenuation or reversal of progression of said microorganism infection. In some embodiments, ibudilast or a pharmaceutically acceptable salt thereof is administered at a daily dosage amount ranging from about 0.1 mg to 720 mg daily, from about 30 mg to 720 mg daily, from about 60 mg to 600 mg daily, or from about 100 mg to 480 mg daily. In some non-claimed embodiments, suppressing MDSCs includes preventing the migration of MDSCs into one or more tumor cells of the patient. In some embodiments, suppressing MDSCs includes reducing MDSC count in the patient. In some embodiments, suppressing MDSCs includes reducing the activity of the MDSCs.

Ibudilast administration may be accomplished through various modes of delivery of ibudilast comprising formulations. Preferred methods of delivery of ibudilast-based therapeutic formulations include systemic and localized delivery. Such routes of administration include but are not limited to, oral, intra-arterial, intrathecal, intraspinal, intramuscular, intraperitoneal, intranasal, and inhalation routes.

More particularly, an ibudilast-based formulation of the present disclosure may be administered for therapy by any suitable route, including without limitation, oral, rectal, nasal, topical (including transdermal, aerosol, buccal and sublingual), vaginal, parenteral (including subcutaneous, intravenous, intramuscular, and intradermal), intrathecal, and pulmonary. In some embodiments, the ibudilast-based formulation is administered orally. In some embodiments, the ibudilast-based formulation is administered through an injection. The preferred route will, of course, vary with the condition and age of the recipient, the particular syndrome being treated, and the specific combination of drugs employed.

In some embodiments, the ibudilast or pharmaceutically acceptable salt thereof is administered orally. In some embodiments, the ibudilast or pharmaceutically acceptable salt thereof is administered through an injection.

In a non-claimed aspect, an ibudilast composition of the present disclosure, when comprising more than one active agent, may be administered as a single combination composition comprising a combination of ibudilast and at least one additional active agent effective in suppressing myeloid-derived suppressor cells (MDSCs), reducing immune suppression, reducing regulatory T-cell count and increasing CD4+ T-cell count in cancer patients. In terms of patient compliance and ease of administration, such an approach is preferred, since patients are often averse to taking multiple pills or dosage forms, often multiple times daily, over the duration of treatment. Alternatively, albeit less preferably, the combination of the disclosure is administered as separate dosage forms. In instances in which the drugs comprising the therapeutic composition of the disclosure are administered as separate dosage forms and coadministration is required, ibudilast and each of the additional active agents may be administered simultaneously, sequentially in any order, or separately.

### Dosages

Therapeutic amounts can be empirically determined and will vary with the particular condition being treated, the subject, and the efficacy and toxicity of each of the active agents contained in the composition. The actual dose to be administered will vary depending upon the age, weight, and general condition of the subject as well as the severity of the condition being treated, the judgment of the health care professional, and particular combination being administered.

Therapeutically effective amounts can be determined by those skilled in the art, and will be adjusted to the requirements of each particular case. Generally, a therapeutically effective amount of ibudilast or pharmaceutically acceptable salt thereof will range from a total daily dosage of about 0.1 mg/day to 720 mg/day, about 60-600 mg/day, or about 100-480 mg/day, or more preferably, in an amount between about 1-240 mg/day, about 30-240 mg/day, about 30-200 mg/day, about 30-120 mg/day, about 1-120 mg/day, about 50-150 mg/day, about 60-150 mg/day, about 60-120 mg/day, or about 60-100 mg/day, administered as either a single dosage or as multiple dosages. In some embodiments, the therapeutically effective amount of ibudilast or pharmaceutically acceptable salt thereof is from about 30-200 mg/day, administered as either a single dosage or as multiple dosages. In some embodiments, multiple dosages include two, three, or four doses per day.

Preferred dosage amounts include dosages greater than about 20 mg BID or TID. That is to say, a preferred dosage amount is greater than about 30 mg/day, 60 mg/day, 90 mg/day, 120 mg/day, 150 mg/day, 180 mg/day, 210 mg/day, 240 mg/day, 270 mg/day, 300 mg/day, 360 mg/day, 400 mg/day, 440 mg/day, 480 mg/day, 520 mg/day, 580 mg/day, 600 mg/day, 620 mg/day, 640 mg/day, 680 mg/day, and 720 mg/day or more.

In some embodiments, the therapeutically effective amount of ibudilast or pharmaceutically acceptable salt thereof is at least 30 mg/day, at least 40 mg/day, at least 50 mg/day, at least 60 mg/day, at least 70 mg/day, at least 80 mg/day, at least 90 mg/day, at least 100 mg/day, at least 110 mg/day, at least 120 mg/day, at least 130 mg/day, at least 140 mg/day, at least 150 mg/day, at least 160 mg/day, at least 170 mg/day, at least 180 mg/day, at least 190 mg/day, at least 200 mg/day, at least 225 mg/day, at least 250 mg/day, at least 275 mg/day, at least 300 mg/day, at least 325 mg/day, at least 350 mg/day, at least 375 mg/day, at least 400 mg/day, at least 425 mg/day, at least 450 mg/day, at least 475 mg/day, at least 500 mg/day, at least 525 mg/day, at least 550 mg/day, at least 575 mg/day, at least 600 mg/day, at least 625 mg/day, at least 650 mg/day, at least 675 mg/day, at least 700 mg/day, or at least 720 mg/day. In some embodiments, the therapeutically effective amount of ibudilast or pharmaceutically acceptable salt thereof is at least 60 mg/day. In some embodiments, the therapeutically effective amount of ibudilast or pharmaceutically acceptable salt thereof is at least 100 mg/day.

Depending upon the dosage amount and precise condition to be treated, administration can be one, two, three, or four times daily for a time course of one day to several days, weeks, months, and even years, and may even be for the life of the patient. Illustrative dosing regimens will last a period of at least about a week, from about 1-4 weeks, from 1-3 months, from 1-6 months, from 1-52 weeks, from 1-24 months, or longer. In some embodiments, the ibudilast or the pharmaceutically acceptable salt thereof is administered for three months or less. In some embodiments, the ibudilast or the pharmaceutically acceptable salt thereof is administered for at least three months. In some embodiments, the ibudilast or the pharmaceutically acceptable salt thereof is administered for at least six months. In some embodiments, the ibudilast or the pharmaceutically acceptable salt thereof is administered for at least one year. In some embodiments, the ibudilast or the pharmaceutically acceptable salt thereof is administered for at least two years. In some embodiments, the ibudilast or the pharmaceutically acceptable salt thereof is administered for at least three years.

In some embodiments, the therapeutically effective amount of ibudilast or the pharmaceutically acceptable salt thereof is administered in a single dosage per day. In some embodiments, the therapeutically effective amount of ibudilast or the pharmaceutically acceptable salt thereof is administered in two dosages per day. In some embodiments, the therapeutically effective amount of ibudilast or the pharmaceutically acceptable salt thereof is administered in three dosages per day. In some embodiments, the therapeutically effective amount of ibudilast or the pharmaceutically acceptable salt thereof is administered in four dosages per day.

In some embodiments, the ibudilast or pharmaceutically acceptable salt thereof is administered at least once daily. In some embodiments, the ibudilast or pharmaceutically acceptable salt thereof is administered at least twice daily.

Practically speaking, a unit dose of any given composition of the disclosure or active agent can be administered in a variety of dosing schedules, depending on the judgment of the clinician, needs of the patient, and so forth. The specific dosing schedule will be known by those of ordinary skill in the art or can be determined experimentally using routine methods. Exemplary dosing schedules include, without limitation, administration five times a day, four times a day, three times a day, twice daily, once daily, every other day, three times weekly, twice weekly, once weekly, twice monthly, once monthly, and so forth.

### Formulations

Ibudilast may be administered in a composition of formulation which may optionally contain one or more additional components as described below.

### Excipients/Carriers

In addition to ibudilast or a pharmaceutically acceptable salt thereof, the compositions of the disclosure for suppressing myeloid-derived suppressor cells (MDSCs), reducing immune suppression, reducing regulatory T-cell count and increasing CD4+ T-cell count in cancer patients may further comprise one or more pharmaceutically acceptable excipients or carriers. Exemplary excipients include, without limitation, polyethylene glycol (PEG), PEG 400, (2-Hydroxypropyl)-β-cyclodextrin, hydrogenated castor oil (HCO), cremophors, carbohydrates, starches (e.g., corn starch), inorganic salts, antimicrobial agents, antioxidants, binders/fillers, surfactants, lubricants (e.g., calcium or magnesium stearate), glidants such as talc, disintegrants, diluents, buffers, acids, bases, film coats, combinations thereof, and the like.

A composition of the disclosure may include one or more carbohydrates such as a sugar, a derivatized sugar such as an alditol, aldonic acid, an esterified sugar, and/or a sugar polymer. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like.

Also suitable for use in the compositions of the disclosure are potato and corn-based starches such as sodium starch glycolate and directly compressible modified starch.

Further representative excipients include inorganic salt or buffers such as citric acid, sodium chloride, potassium chloride, sodium sulfate, potassium nitrate, sodium phosphate monobasic, sodium phosphate dibasic, and combinations thereof.

A composition of the disclosure may also contain one or more antioxidants. Antioxidants are used to prevent oxidation, thereby preventing the deterioration of the drug(s) or other components of the preparation. Suitable antioxidants for use in the present disclosure include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

Additional exemplary excipients include surfactants such as polysorbates, e.g., "Tween 20" and "Tween 80," and pluronics such as F68 and F88 (both of which are available from BASF, Mount Olive, N.J.), sorbitan esters, lipids (e.g., phospholipids such as lecithin and other phosphatidylcholines, and phosphatidylethanolamines), fatty acids and fatty esters, steroids such as cholesterol, and chelating agents, such as EDTA, zinc and other such suitable cations.

Further, a composition of the disclosure may optionally include one or more acids or bases. Non-limiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Non-limiting examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof.

The amount of any individual excipient in the composition will vary depending on the role of the excipient, the dosage requirements of the active agent components, and particular needs of the composition. Typically, the optimal amount of any individual excipient is determined through routine experimentation, i.e., by preparing compositions containing varying amounts of the excipient (ranging from low to high), examining the stability and other parameters, and then determining the range at which optimal performance is attained with no significant adverse effects.

Generally, however, the excipient will be present in the composition in an amount of about 1% to about 99% by weight, preferably from about 5% to about 98% by weight, more preferably from about 15% to about 95% by weight of the excipient. In general, the amount of excipient present in an ibudilast composition of the disclosure is selected from the following: at least about 2%, 5%, 10%, 15%, 20%, 25%, 30%, 35%, 40%, 45%, 50%, 55%, 60%, 65%, 70%, 75%, 80%, 85%, 90%, or even 95% by weight.

These foregoing pharmaceutical excipients along with other excipients are described in "Remington: The Science & Practice of Pharmacy", 19th ed., Williams & Williams, (1995), the "Physician's Desk Reference", 52.sup.nd ed., Medical Economics, Montvale, N.J. (1998), and Kibbe, A. H., Handbook of Pharmaceutical Excipients, 3.sup.rd Edition, American Pharmaceutical Association, Washington, D.C., 2000.

### Other Actives (not part of the present invention)

A formulation (or kit) in accordance with the disclosure may contain, in addition to ibudilast or a pharmaceutically acceptable salt thereof, one or more other therapeutic active agents effective in suppressing myeloid-derived suppressor cells (MDSCs), reducing immune suppression, reducing regulatory T-cell count and increasing CD4+ T-cell count in cancer patients. In some embodiments, the one or more other therapeutic agents comprise a phosphodiesterase-3 inhibitor. In some embodiments, the one or more other therapeutic agents comprise a phosphodiesterase-4 inhibitor. In some embodiments, the one or more other therapeutic agents comprise a macrophage inhibitory factor inhibitor. In some embodiments, the one or more other therapeutic agents comprise laquinimod. In a preferred embodiment, the one or more other therapeutic agents possess a mechanism of action different from ibudilast.

Preferably, the one or more other therapeutic agent is one that possesses a mechanism of action different from that of ibudilast. Such active ingredients can be found listed in the FDA's Orange Book, Goodman & Gilman The Pharmacological Basis of Therapeutics, J. Griffith Hardman, L. L. Limbird, A. Gilman, 11th Ed., 2005, The Merck Manual, 18th edition, 2007, and The Merck Manual of Medical Information 2003.

The dosage amounts provided above are meant to be merely guidelines; the precise amount of a secondary active agent to be administered during combination therapy with ibudilast or the pharmaceutically acceptable salt thereof will, of course, be adjusted accordingly and will depend upon factors such as intended patient population, the particular progressive neuropathic disease symptom or condition to be treated, potential synergies between the active agents administered, and the like, and will readily be determined by one skilled in the art based upon the guidance provided herein.

### Sustained Delivery Formulations

Preferably, the compositions are formulated in order to improve stability and extend the half-life of ibudilast or the pharmaceutically acceptable salt thereof. For example, ibudilast or the pharmaceutically acceptable salt thereof may be delivered in a controlled or extended-release formulation. Controlled or extended-release formulations are prepared by incorporating ibudilast or the pharmaceutically acceptable salt thereof into a carrier or vehicle such as liposomes, nonresorbable impermeable polymers such as ethylenevinyl acetate copolymers and Hytrel^{®} copolymers, swellable polymers such as hydrogels, or resorbable polymers such as collagen and certain polyacids or polyesters such as those used to make resorbable sutures. Additionally, ibudilast or the pharmaceutically acceptable salt thereof can be encapsulated, adsorbed to, or associated with, particulate carriers. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; and McGee et al., J. Microencap. (1996).

Extended release polymers suitable for this purpose are known in the art and include hydrophobic polymers such as cellulose ethers. Non-limiting examples of suitable cellulose ethers include ethyl cellulose, cellulose acetate and the like; polyvinyl esters such as polyvinyl acetate, polyacrylic acid esters, methacrylic and acrylate polymers (pH-independent types); high molecular weight polyvinyl alcohols and waxes such as fatty acids and glycerides, methacrylic acid ester neutral polymers, polyvinyl alcohol-maleic anhydride copolymers and the like; ethylacrylate-methylmethacrylate copolymers; aminoalkyl methacrylate copolymers; and mixtures thereof.

### Delivery Forms

The ibudilast or pharmaceutically acceptable salt thereof compositions described herein encompass all types of formulations, and in particular, those that are suited for systemic or intrathecal administration. Oral dosage forms include tablets, lozenges, capsules, syrups, oral suspensions, emulsions, granules, and pellets. In some embodiments, the oral dosage form is a tablet. In some embodiments, the tablet is an extended release tablet. In some embodiments, the oral dosage form is a capsule. In some embodiments, the capsule is an extended release capsule.

Alternative formulations include aerosols, transdermal patches, gels, creams, ointments, suppositories, powders or lyophilates that can be reconstituted, as well as liquids. Examples of suitable diluents for reconstituting solid compositions, e.g., prior to injection, include bacteriostatic water for injection, dextrose 5% in water, phosphate-buffered saline, Ringer's solution, saline, sterile water, deionized water, and combinations thereof. With respect to liquid pharmaceutical compositions, solutions and suspensions are envisioned. Preferably, an ibudilast or pharmaceutically acceptable salt thereof composition of the disclosure is one suited for oral administration.

In turning now to oral delivery formulations, tablets can be made by compression or molding, optionally with one or more accessory ingredients or additives. Compressed tablets are prepared, for example, by compressing in a suitable tabletting machine, the active ingredients in a free-flowing form such as a powder or granules, optionally mixed with a binder (e.g., povidone, gelatin, hydroxypropylmethyl cellulose), lubricant, inert diluent, preservative, disintegrant (e.g., sodium starch glycolate, cross-linked povidone, cross-linked sodium carboxymethyl cellulose) and/or surface-active or dispersing agent.

Molded tablets are made, for example, by molding in a suitable tabletting machine, a mixture of powdered compounds moistened with an inert liquid diluent. The tablets may optionally be coated or scored, and may be formulated so as to provide slow or controlled release of the active ingredients, using, for example, hydroxypropylmethyl cellulose in varying proportions to provide the desired release profile. Tablets may optionally be provided with a coating, such as a thin film, sugar coating, or an enteric coating to provide release in parts of the gut other than the stomach. Processes, equipment, and toll manufacturers for tablet and capsule making are well-known in the art.

Formulations for topical administration in the mouth include lozenges comprising the active ingredients, generally in a flavored base such as sucrose and acacia or tragacanth and pastilles comprising the active ingredients in an inert base such as gelatin and glycerin or sucrose and acacia.

A pharmaceutical composition for topical administration may also be formulated as an ointment, cream, suspension, lotion, powder, solution, paste, gel, spray, aerosol or oil.

Alternatively, the formulation may be in the form of a patch (e.g., a transdermal patch) or a dressing such as a bandage or adhesive plaster impregnated with active ingredients and optionally one or more excipients or diluents. Topical formulations may additionally include a compound that enhances absorption or penetration of the ingredients through the skin or other affected areas, such as dimethylsulfoxidem bisabolol, oleic acid, isopropyl myristate, and D-limonene, to name a few.

For emulsions, the oily phase is constituted from known ingredients in a known manner. While this phase may comprise merely an emulsifier (otherwise known as an emulgent), it desirably comprises a mixture of at least one emulsifier with a fat and/or an oil. Preferably, a hydrophilic emulsifier is included together with a lipophilic emulsifier that acts as a stabilizer. Together, the emulsifier(s) with or without stabilizer(s) make up the so-called emulsifying wax, and the wax together with the oil and/or fat make up the so-called emulsifying ointment base which forms the oily dispersed phase of cream formulations. Illustrative emulgents and emulsion stabilizers include Tween 60, Span 80, cetostearyl alcohol, myristyl alcohol, glyceryl monostearate and sodium lauryl sulfate.

Formulations for rectal administration are typically in the form of a suppository with a suitable base comprising, for example, cocoa butter or a salicylate.

Formulations suitable for vaginal administration generally take the form of a suppository, tampon, cream, gel, paste, foam or spray.

Formulations suitable for nasal administration, wherein the carrier is a solid, include a coarse powder having a particle size, for example, in the range of about 20 to about 500 microns. Such a formulation is typically administered by rapid inhalation through the nasal passage, e.g., from a container of the powder held in proximity to the nose. Alternatively, a formulation for nasal delivery may be in the form of a liquid, e.g., a nasal spray or nasal drops.

Aerosolizable formulations for inhalation may be in dry powder form (e.g., suitable for administration by a dry powder inhaler), or, alternatively, may be in liquid form, e.g., for use in a nebulizer. Nebulizers for delivering an aerosolized solution include the AERx^{®} (Aradigm), the Ultravent^{®} (Mallinkrodt), and the Acorn II^{®} (Marquest Medical Products). A composition of the disclosure may also be delivered using a pressurized, metered dose inhaler (MDI), e.g., the Ventolin^{®} metered dose inhaler, containing a solution or suspension of a combination of drugs as described herein in a pharmaceutically inert liquid propellant, e.g., a chlorofluorocarbon or fluorocarbon.

Formulations suitable for parenteral administration include aqueous and non-aqueous isotonic sterile solutions suitable for injection, as well as aqueous and non-aqueous sterile suspensions.

Parenteral formulations of the disclosure are optionally contained in unit-dose or multi-dose sealed containers, for example, ampoules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the types previously described.

A formulation of the disclosure may also be an extended release formulation, such that each of the drug components is released or absorbed slowly over time, when compared to a non-sustained release formulation. Sustained release formulations may employ pro-drug forms of the active agent, delayed-release drug delivery systems such as liposomes or polymer matrices, hydrogels, or covalent attachment of a polymer such as polyethylene glycol to the active agent.

In addition to the ingredients particularly mentioned above, the formulations of the disclosure may optionally include other agents conventional in the pharmaceutical arts and particular type of formulation being employed, for example, for oral administration forms, the composition for oral administration may also include additional agents as sweeteners, thickeners or flavoring agents.

### Kits (not part of the present invention)

Also provided herein is a kit containing at least one combination composition of the disclosure, accompanied by instructions for use.

For example, in instances in which each of the drugs themselves are administered as individual or separate dosage forms, the kit comprises ibudilast in addition to each of the drugs making up the composition of the disclosure, along with instructions for use. The drug components may be packaged in any manner suitable for administration, so long as the packaging, when considered along with the instructions for administration, clearly indicates the manner in which each of the drug components is to be administered.

For example, for an illustrative kit comprising ibudilast and gabapentin, the kit may be organized by any appropriate time period, such as by day. As an example, for Day 1, a representative kit may comprise unit dosages of each of ibudilast and gabapentin. If each of the drugs is to be administered twice daily, then the kit may contain, corresponding to Day 1, two rows of unit dosage forms of each of ibudilast and gabapentin, along with instructions for the timing of administration. Alternatively, if one or more of the drugs differs in the timing or quantity of unit dosage form to be administered in comparison to the other drug members of the combination, then such would be reflected in the packaging and instructions. Various embodiments according to the above may be readily envisioned, and would of course depend upon the particular combination of drugs, in addition to ibudilast, employed for treatment, their corresponding dosage forms, recommended dosages, intended patient population, and the like. The packaging may be in any form commonly employed for the packaging of pharmaceuticals, and may utilize any of a number of features such as different colors, wrapping, tamper-resistant packaging, blister packs, desiccants, and the like.

It is to be understood that while the disclosure has been described in conjunction with preferred specific embodiments, the foregoing description as well as the examples that follow are intended to illustrate and not limit the scope of the disclosure. Other aspects, advantages and modifications within the scope of the disclosure will be apparent to those skilled in the art to which the disclosure pertains.

The following examples are given for the purpose of illustrating various embodiments of the disclosure and are not meant to limit the present disclosure in any fashion. One skilled in the art will appreciate readily that the present disclosure is well adapted to carry out the objects and obtain the ends and advantages mentioned, as well as those objects, ends and advantages inherent herein. The present examples, along with the methods described herein are presently representative of embodiments and are exemplary, and are not intended as limitations on the scope of the disclosure. Changes therein and other uses which are encompassed within the spirit of the disclosure as defined by the scope of the claims will occur to those skilled in the art.

### EXAMPLES

### Example 1: Ibudilast treated MDSCs' effect on T-cell proliferation in vitro

### Preparation of MDSCs

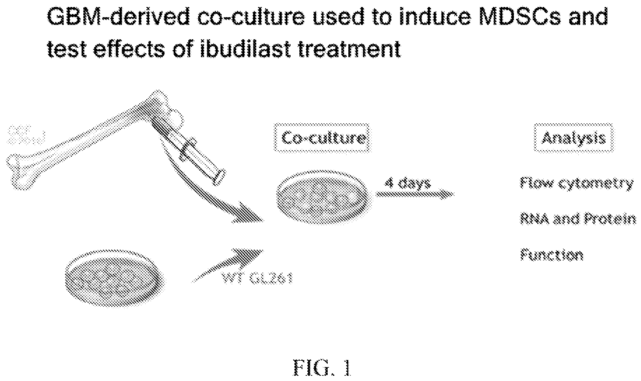

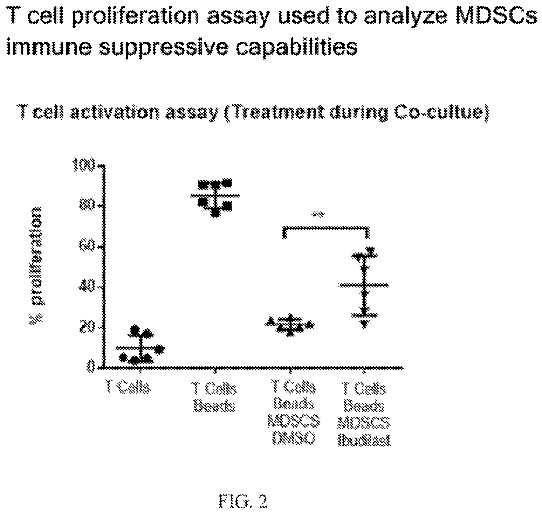

MDSCs were prepared through a co-culture process illustrated in FIG. 1. Glioma cell line GL261 is cultured together with freshly isolated bone marrow for 4 days. During this 4-day process the glioma cell line initiates subsets of the naive monocytes within the bone marrow to become both monocytic MDSCs (M-MDSCs) and granulocytic MDSCs (G-MDSCs) distinguished via flow cytometry using CD11b, Ly6C, and GR1. The GR1+ population was harvested at day 4 via magnetic bead separation. Once isolated the MDSCs were combined with freshly isolated splenic T-cells which were labeled with CFSE and activated using CD3/28 activation beads. The T-cell proliferation was then determined 3 days after being combined with MDSCs by measuring the fluorescence intensity of each CD3 T-cell after 3 days of being cultured with MDSCs (Figure 2). Analysis revealed that MDSCs treated with ibudilast effectively reduced the suppressive abilities of MDSCs in n=6 mice in 2 separate experiments (P=0.001) as shown in FIG. 2.

### Reference

### Example 2: Effect of ibudilast and AV1013 on MDSCs and T-cells in blood, tumor, marrow and spleen in vivo

Ibudilast was tested *in vivo* using the mouse model of glioma GL261. Cells were implanted on day 0 and then a 5-day engraftment period was used to ensure that the treatment being tested was performing by inhibiting tumor growth and not engraftment. After 5 days the mice were intraperitoneally injected daily with ibudilast at 50 mg/kg in a vehicle consisting of PEG 400 and (2-hydroxypropyl)-β-cyclodextrin to enhance solubility with low toxicity or alternatively mice were treated with AV1013. At day 19 post injection, 3 mice from each group (ibudilast, AV1013 or vehicle) were sacrificed for flow cytometry analysis of MDSCs, T-cells, and NK cells in the tumor, blood, spleen, and bone marrow.

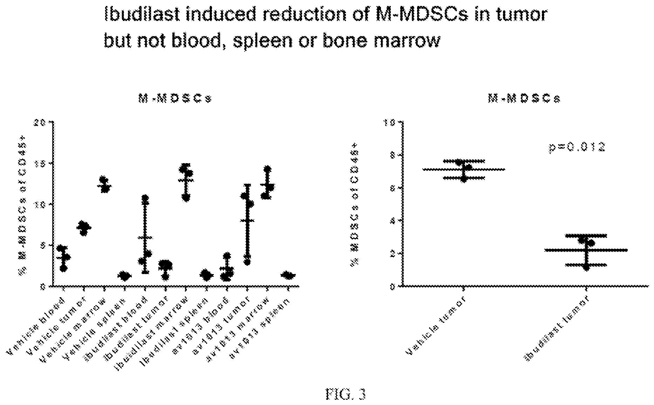

The results of this study revealed that monocytic MDSCs (M-MDSCs) were reduced in the tumor of ibudilast-treated mice, but were not altered in the blood, marrow or spleen (FIG. 3). AV1013 treated mice showed no significant suppression of M-MDSCs in the tumor versus vehicle-treated mice.

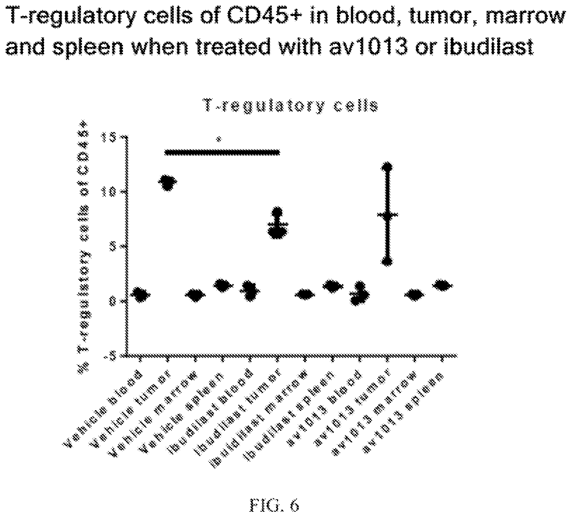

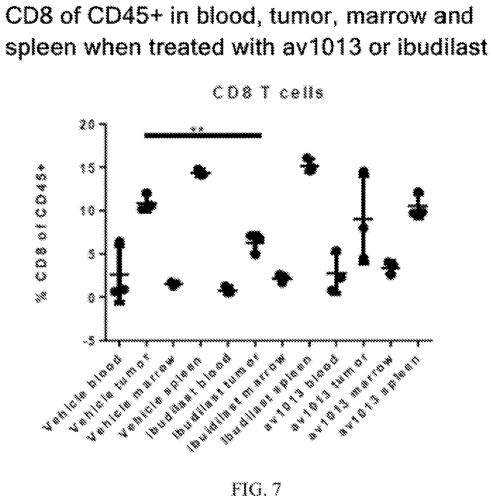

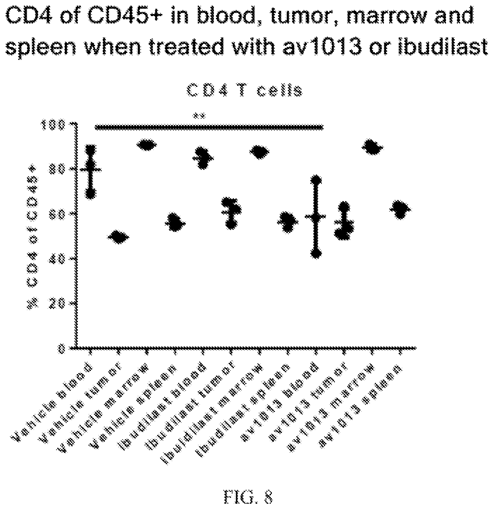

Additionally, the immune suppressive T-regulatory cells were also reduced only in the tumors of the ibudilast-treated mice demonstrating an overall reduction of immune suppression in the tumor (FIG. 6). When CD4 and CD8 T-cells were examined, it was found that the CD4 T-cells were increased, while CD8 T-cells were decreased (FIG. 8 and 7, respectively).

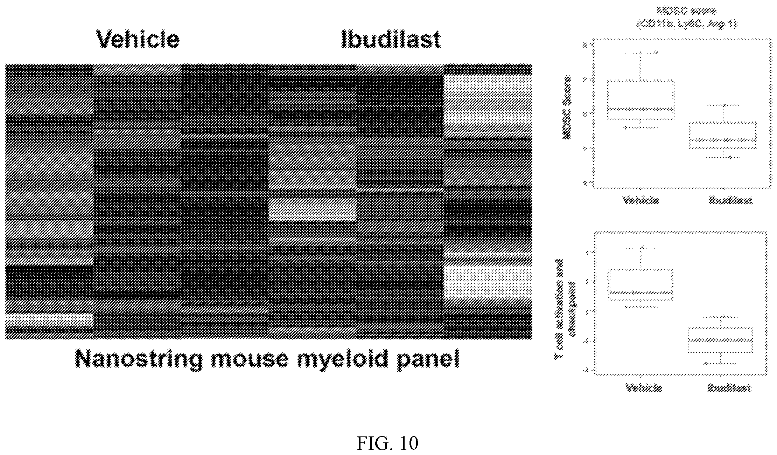

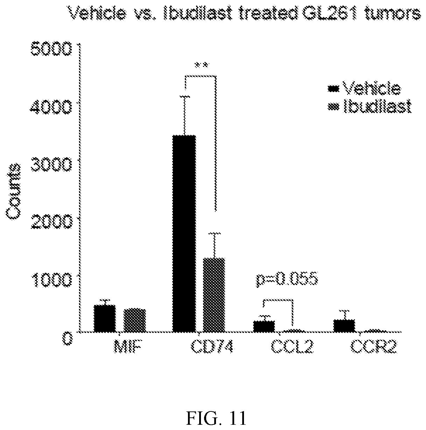

Further analysis was conducted on the mRNA expression of CD45+ cells isolated from vehicle- and ibudilast-treated tumors. Overall reductions in myeloid genes were indicated upon ibudilast treatment (FIG. 10). MIF was not reduced, but CD74 was significantly reduced, along with MCP-1 (CCL2) and its receptor CCR2 (FIG. 11). Overall, these reductions indicate that the MIF/CD74 signaling pathway was inhibited.

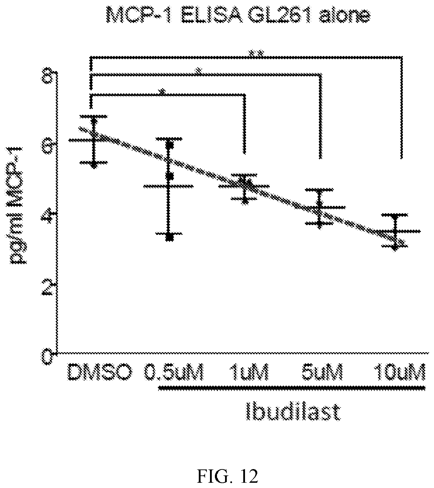

*In vitro* ibudilast treatment of co-culture for 24 h had led to reduced GLP261 secretion MCP-1 (CCL2) in a dose-dependent manner indicating inhibitor of the MIF/CD74 signaling axis (FIG. 12).

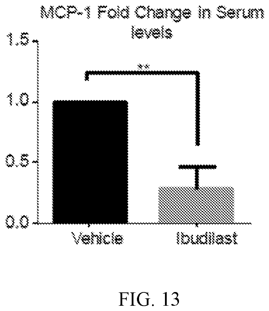

Serum from these animals at day 19 was analyzed with 3 animals in each group. The cytokine levels in treated mice revealed a reduction in MCP-1, which is downstream of MIF/CD74 signaling axis, indicating a reduction in MIF signaling in the ibudilast-treated mice (FIG. 13).

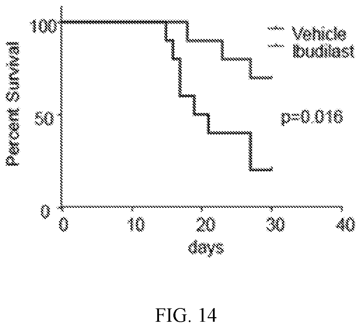

The median survival times for vehicle-treated mice (n=10) was 19 days whereas ibudilast-treated mice (n=10) were undetermined at this point (Log Rank p=0.016, unpaired T-test) (FIG. 14). Significant survival advantages were observed in ibudilast-treated mice compared to mice treated with vehicle.

### Reference

### Example 3: Administration of ibudilast and temozolomide (TMZ) combination in patients with glioblastoma multiforme (GBM)

Patients with GBM are treated via administering ibudilast and TMZ over the course of a 28 day dosing cycle. TMZ is administered on days 1-5 of the 28 day cycle (at a dosage of 100, 150 or 200 mg/(m^{2∗}day)) or on days 1-21 of the 28 day cycle (at a dosage of 75 mg/(m^{2∗}day)). Ibudilast is administered on every day of the dosing cycle. Treatment may comprise consecutive cycles.

Ibudilast is administered at the same dose throughout a dose cycle. Dose cohorts possible are 60 mg per day (30 mg b.i.d.) or 100 mg (50 mg each b.i.d.). Ibudilast is administered at a dosage of 40 mg per day (20 mg b.i.d.) if the 60 mg per day dose is not well tolerated.

Patient blood is collected before and after treatment for evaluation of MDSCs, regulatory T-cells, and CD4+ T-cells.

Progression free survival and overall survival of the patients are assessed.

### Reference

### Example 4: Administration of ibudilast in patients with glioblastoma multiforme (GBM)

Patients with GBM are treated via administering ibudilast over the course of a 28 day dosing cycle. Ibudilast is administered on every day of the dosing cycle. Treatment may comprise consecutive cycles.

Ibudilast is administered at the same dose throughout a dose cycle. Dose cohorts possible are 60 mg per day (30 mg b.i.d.) or 100 mg (50 mg each b.i.d.). Ibudilast is administered at a dosage of 40 mg per day (20 mg b.i.d.) if the 60 mg per day dose is not well tolerated.

Patient blood is collected before and after treatment for evaluation of MDSCs, regulatory T-cells, and CD4+ T-cells.

Progression free survival and overall survival of the patients are assessed.

### Reference

### Example 5: Administration of ibudilast in patients with breast cancer

Patients with breast cancer are treated via administering ibudilast over the course of a 28 day dosing cycle. Ibudilast is administered on every day of the dosing cycle. Treatment may comprise consecutive cycles.

Ibudilast is administered at the same dose throughout a dose cycle. Dose cohorts possible are 60 mg per day (30 mg b.i.d.) or 100 mg (50 mg each b.i.d.). Ibudilast is administered at a dosage of 40 mg per day (20 mg b.i.d.) if the 60 mg per day dose is not well tolerated.

Patient blood is collected before and after treatment for evaluation of MDSCs, regulatory T-cells, and CD4+ T-cells.

Progression free survival and overall survival of the patients are assessed.

### Reference

### Example 6: Administration of ibudilast in patients with colon cancer

Patients with colon cancer are treated via administering ibudilast over the course of a 28 day dosing cycle. Ibudilast is administered on every day of the dosing cycle. Treatment may comprise consecutive cycles.

Ibudilast is administered at the same dose throughout a dose cycle. Dose cohorts possible are 60 mg per day (30 mg b.i.d.) or 100 mg (50 mg each b.i.d.). Ibudilast is administered at a dosage of 40 mg per day (20 mg b.i.d.) if the 60 mg per day dose is not well tolerated.

Patient blood is collected before and after treatment for evaluation of MDSCs, regulatory T-cells, and CD4+ T-cells.

Progression free survival and overall survival of the patients are assessed.

### Example 7: Impact of MIF inhibition on MDSC generation and function

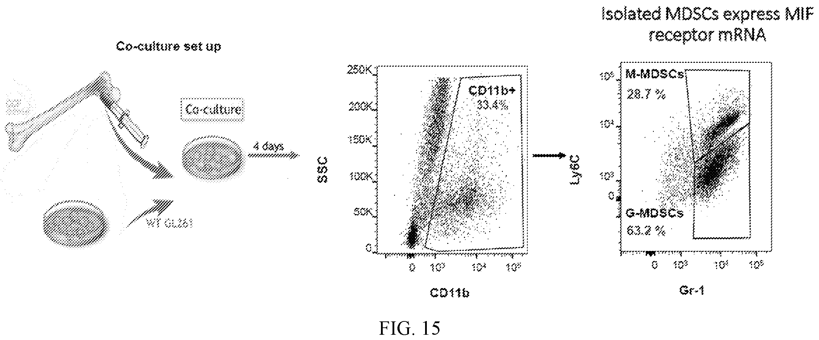

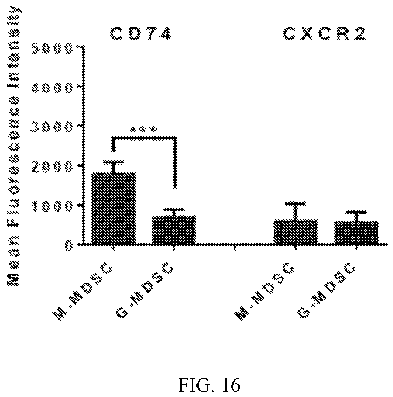

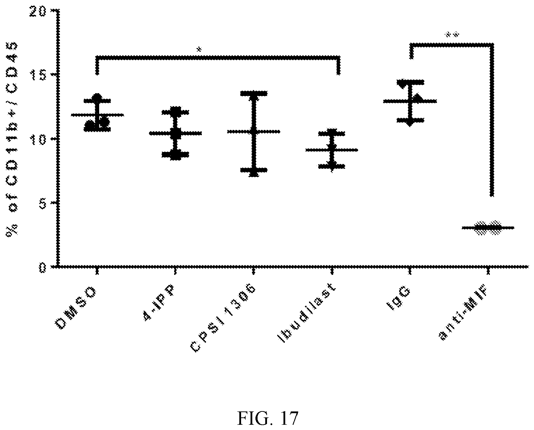

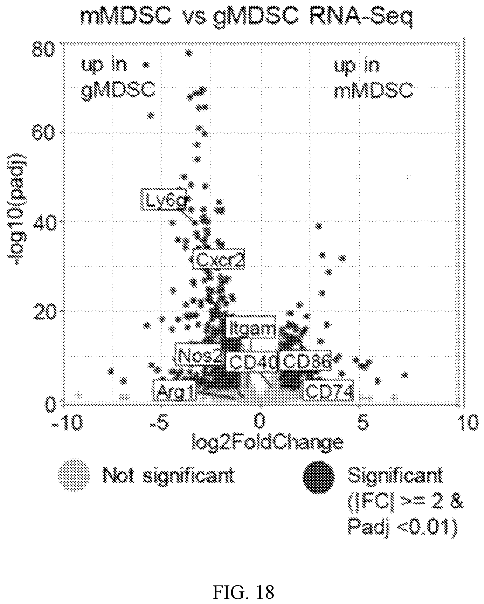

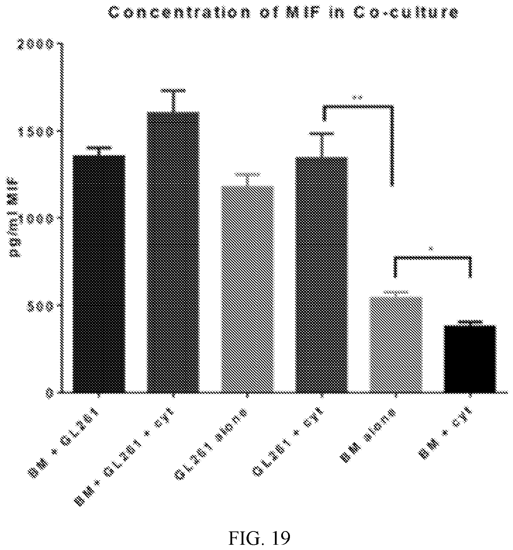

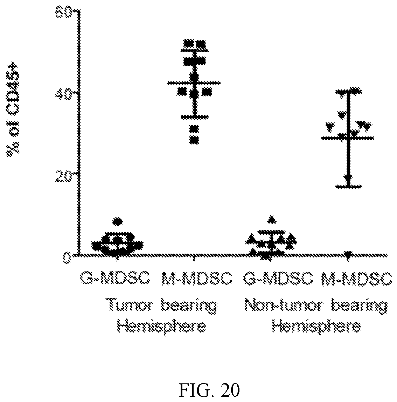

As described in Example 1, a co-culture system can produce monocytic MDSCs (M-MDSCs) and granulocytic MDSCs (G-MDSCs) (FIG. 15). To investigate how each subset differed in their MIF signaling, the primary MIF receptors, CD74 and CXCR2, were examined via flow cytometry staining on the surface of M-MDSCs and G-MSCs (FIG. 16). This analysis demonstrated that CD74 was the primary MIF receptor on M-MDSCs, while G-MDSCSs had relatively low CD74 and CXCR2 expression. When MIF/CD74 interaction inhibitors were examined with the co-culture system, ibudilast demonstrated an ability to reduce M-MDSC generation (FIG. 17). RNA sequencing of M-MDSCs, G-MDSCs and CD11b+ non MDSCs also revealed that each population was distinct at the transcriptional level from one another, and confirmed that CD74 was primarily expressed on M-MDSCs (FIG. 18). In order to determine which cells in the co-culture were the primary producers of MIF, ELISA analysis was performed of the co-culture system components separate and combined after 24 hours. ELISA analysis identified the primary source of MIF to be the glioma cell line, GL261, and not from the bone marrow derived cells (FIG. 19). Lastly, an *in vivo* experiment where MDSC subpopulations were quantified in the tumors of 10 GL261 bearing mice at day 19 post tumor implantation was performed (FIG. 20). These results identified that the M-MDSC population was increased in the tumor bearing hemisphere and contralateral hemisphere of the brain, making M-MDSCs the primary population of interest for immune suppression targeting in GBM (FIG. 20).

## Claims

1. Ibudilast, or a pharmaceutical salt thereof, for use in a treatment for suppressing myeloid-derived suppressor cells (MDSCs) in a patient diagnosed with microorganism infection or suffering therefrom, preferably wherein suppression of MDSCs reduces immune suppression in the patient, more preferably wherein suppression of MDSCs increases CD4 T-cell count in the patient; and wherein ibudilast, or the pharmaceutically acceptable salt thereof, is the only active agent administered to the patient.

2. Ibudilast, or a pharmaceutical salt thereof, for use in a treatment for: reducing immune suppression; reducing regulatory T-cell count; or increasing CD4+ T-cell count, in a patient diagnosed with microorganism infection or suffering therefrom; wherein ibudilast, or the pharmaceutically acceptable salt thereof, is the only active agent administered to the patient.

3. Ibudilast, or a pharmaceutical salt thereof, for a use according to claim 1 or claim 2, wherein the microorganism infection is caused by virus, bacteria, fungus, or any combination of two or more thereof.

4. Ibudilast, or a pharmaceutical salt thereof, for a use according to any one of claims 1 to 3, wherein ibudilast, or the pharmaceutically acceptable salt thereof, is administered for at least 3 months, preferably for at least six months, more preferably for at least one year, even more preferably for at least two years.

5. Ibudilast, or a pharmaceutical salt thereof, for a use according to any one of claims 1 to 4, wherein ibudilast, or the pharmaceutically acceptable salt thereof, is administered at least once daily, optionally, orally.

6. Ibudilast, or a pharmaceutical salt thereof, for a use according to any one of claims 1 to 5, wherein the therapeutically effective amount of ibudilast, or the pharmaceutically acceptable salt thereof, is from 0.1 mg to 720 mg per day, for example: at least 30 mg/day; from 30 mg to 200 mg per day; 60 mg to 600 mg daily; or 100 mg to 480 mg daily.

7. Ibudilast, or a pharmaceutical salt thereof, for a use according to any one of claims 1 to 5, wherein the therapeutically effective amount of ibudilast, or the pharmaceutically acceptable salt thereof, is selected from the group consisting of 30 mg/day, 60 mg/day, 90 mg/day, 100 mg/day, 120 mg/day, 150 mg/day, 180 mg/day, 210 mg/day, 240 mg/day, 270 mg/day, 300 mg/day, 360 mg/day, 400 mg/day, 440 mg/day, 480 mg/day, 520 mg/day, 580 mg/day, 600 mg/day, 620 mg/day, 640 mg/day, 680 mg/day, and 720 mg/day.

8. Ibudilast, or a pharmaceutical salt thereof, for a use according to any one of claims 1 to 7, wherein the therapeutically effective amount is administered as a single dose or is divided into two, three, or four doses, or wherein ibudilast is administered continually.

## Patentansprüche

1. Ibudilast oder ein pharmazeutisches Salz davon zur Verwendung bei einer Behandlung zum Unterdrücken myeloider Suppressorzellen (MDSCs) bei einem Patienten, bei dem eine Mikroorganismusinfektion diagnostiziert wurde oder der daran leidet, wobei vorzugsweise die Unterdrückung von MDSCs die Immunsuppression bei dem Patienten verringert, wobei besonders bevorzugt die Unterdrückung von MDSCs die CD4-T-Zellzahl bei dem Patienten erhöht; und wobei Ibudilast oder das pharmazeutisch verträgliche Salz davon der einzige Wirkstoff ist, der dem Patienten verabreicht wird.

2. Ibudilast oder ein pharmazeutisches Salz davon zur Verwendung bei einer Behandlung zum: Reduzieren der Immunsuppression; Reduzieren der regulatorischen T-Zellzahl; oder Erhöhen der CD4+ T-Zellzahl bei einem Patienten, bei dem eine Mikroorganismusinfektion diagnostiziert wurde oder der daran leidet; wobei Ibudilast oder das pharmazeutisch verträgliche Salz davon der einzige Wirkstoff ist, der dem Patienten verabreicht wird.

3. Ibudilast oder ein pharmazeutisches Salz davon für eine Verwendung nach Anspruch 1 oder Anspruch 2, wobei die Mikroorganismusinfektion durch Viren, Bakterien, Fungus oder eine Kombination von zwei oder mehreren davon verursacht wird.

4. Ibudilast oder ein pharmazeutisches Salz davon für eine Verwendung nach einem der Ansprüche 1 bis 3, wobei Ibudilast oder das pharmazeutisch verträgliche Salz davon für mindestens 3 Monate, vorzugsweise für mindestens sechs Monate, besonders bevorzugt für mindestens ein Jahr, noch bevorzugter für mindestens zwei Jahre verabreicht wird.

5. Ibudilast oder ein pharmazeutisches Salz davon für eine Verwendung nach einem der Ansprüche 1 bis 4, wobei Ibudilast oder das pharmazeutisch verträgliche Salz davon mindestens einmal täglich, gegebenenfalls oral, verabreicht wird.

6. Ibudilast oder ein pharmazeutisches Salz davon für eine Verwendung nach einem der Ansprüche 1 bis 5, wobei die therapeutisch wirksame Menge an Ibudilast oder dem pharmazeutisch verträglichen Salz davon 0,1 mg bis 720 mg pro Tag beträgt, zum Beispiel: mindestens 30 mg/Tag; 30 mg bis 200 mg pro Tag; 60 mg bis 600 mg täglich; oder 100 mg bis 480 mg täglich.

7. Ibudilast oder ein pharmazeutisches Salz davon für eine Verwendung nach einem der Ansprüche 1 bis 5, wobei die therapeutisch wirksame Menge an Ibudilast oder dem pharmazeutisch verträglichen Salz davon aus der Gruppe ausgewählt ist, die aus Folgendem besteht: 30 mg/Tag, 60 mg/Tag, 90 mg/Tag, 100 mg/Tag, 120 mg/Tag, 150 mg/Tag, 180 mg/Tag, 210 mg/Tag, 240 mg/Tag, 270 mg/Tag, 300 mg/Tag, 360 mg/Tag, 400 mg/Tag, 440 mg/Tag, 480 mg/Tag, 520 mg/Tag, 580 mg/Tag, 600 mg/Tag, 620 mg/Tag, 640 mg/Tag, 680 mg/Tag und 720 mg/Tag.

8. Ibudilast oder ein pharmazeutisches Salz davon für eine Verwendung nach einem der Ansprüche 1 bis 7, wobei die therapeutisch wirksame Menge als Einzeldosis verabreicht wird oder in zwei, drei oder vier Dosen aufgeteilt wird oder wobei Ibudilast kontinuierlich verabreicht wird.

## Revendications

1. Ibudilast, ou un sel pharmaceutique de celui-ci, pour utilisation dans un traitement de suppression de cellules myéloïdes suppressives (MDSC) chez un patient diagnostiqué avec une infection par un micro-organisme ou qui en souffre, de préférence où la suppression des MDSC réduit l'immunosuppression chez le patient, plus préférablement où la suppression des MDSC augmente le nombre de lymphocytes T CD4 chez le patient ; et où l'ibudilast, ou le sel pharmaceutiquement acceptable de celui-ci, est le seul agent actif administré au patient.

2. Ibudilast, ou un sel pharmaceutique de celui-ci, pour utilisation dans un traitement pour : réduire la suppression immunitaire ; réduire le nombre de lymphocytes T régulateurs ; ou augmenter le nombre de lymphocytes T CD4+, chez un patient diagnostiqué avec une infection par un micro-organisme ou qui en souffre ; où l'ibudilast, ou le sel pharmaceutiquement acceptable de celui-ci, est le seul agent actif administré au patient.

3. Ibudilast, ou un sel pharmaceutique de celui-ci, pour utilisation selon la revendication 1 ou la revendication 2, où l'infection par le micro-organisme est provoquée par un virus, une bactérie, un fongus ou toute combinaison de deux ou plus de ceux-ci.

4. Ibudilast, ou un sel pharmaceutique de celui-ci, pour utilisation selon l'une quelconque des revendications 1 à 3, où l'ibudilast, ou le sel pharmaceutiquement acceptable de celui-ci, est administré pendant au moins 3 mois, de préférence pendant au moins six mois, plus préférablement pendant au moins un an, encore plus préférablement pendant au moins deux ans.

5. Ibudilast, ou un sel pharmaceutique de celui-ci, pour utilisation selon l'une quelconque des revendications 1 à 4, où l'ibudilast, ou le sel pharmaceutiquement acceptable de celui-ci, est administré au moins une fois par jour, éventuellement par voie orale.

6. Ibudilast, ou un sel pharmaceutique de celui-ci, pour utilisation selon l'une quelconque des revendications 1 à 5, où la quantité thérapeutiquement efficace d'ibudilast, ou du sel pharmaceutiquement acceptable de celui-ci, est de 0,1 mg à 720 mg/jour, par exemple : au moins 30 mg/jour ; de 30 mg à 200 mg/jour, 60 mg à 600 mg/jour ; ou 100 mg à 480 mg/jour.

7. Ibudilast, ou un sel pharmaceutique de celui-ci, pour utilisation selon l'une quelconque des revendications 1 à 5, où la quantité thérapeutiquement efficace d'ibudilast, ou du sel pharmaceutiquement acceptable de celui-ci, est choisie dans le groupe consistant en 30 mg/jour, 60 mg/jour, 90 mg/jour, 100 mg/jour, 120 mg/jour, 150 mg/jour, 180 mg/jour, 210 mg/jour, 240 mg/jour, 270 mg/jour, 300 mg/jour, 360 mg/jour, 400 mg/jour, 440 mg/jour, 480 mg/jour, 520 mg/jour, 580 mg/jour, 600 mg/jour, 620 mg/jour, 640 mg/jour, 680 mg/jour, et 720 mg/jour.

8. Ibudilast, ou un sel pharmaceutique de celui-ci, pour utilisation selon l'une quelconque des revendications 1 à 7, où la quantité thérapeutiquement efficace est administrée en une dose unique ou est divisée en deux, trois ou quatre doses, ou où l'ibudilast est administré en continu.
